# EUROPÄISCHE PATENTSCHRIFT

(11) **EP 3 886 771 B1**
(45) Veröffentlichungstag und Bekanntmachung des Hinweises auf die Patenterteilung: **28.12.2022**
(21) Anmeldenummer: 19812744.1
(22) Anmeldetag: 26.11.2019
(51) Int. Cl.: A61F 2/78, A61F 5/01, A61F 5/058, A61F 2/50, A61F 2/68, A61F 2/74, A61F 2/80

(54) **HÜLLKÖRPER MIT VERSTEIFUNGSELEMENTEN**
ENVELOPING BODY WITH REINFORCEMENT ELEMENTS
PALETTE EN MATIÈRE PLASTIQUE COMPRENANT DES ÉLÉMENTS DE RENFORCEMENT

(30) Priorität: 27.11.2018 DE 102018129921
(43) Veröffentlichungstag der Anmeldung: 06.10.2021
(73) Patentinhaber: Ottobock SE & Co. KGaA, 37115 Duderstadt (DE)
(72) Erfinder: ENGELBART, Hendryk, 37083 Göttingen (DE); FINKE, Lars Benjamin, 37136 Landolfshausen (DE)
(74) Vertreter: Gramm, Lins & Partner Patent- und Rechtsanwälte PartGmbB
(86) Internationale Anmeldenummer: PCT/EP2019/082596
(87) Internationale Veröffentlichungsnummer: WO 2020/109309

(56) Entgegenhaltungen:
- WO-A1-2013/113332
- WO-A1-2015/112712
- US-A- 6 066 107

## Beschreibung

Die Erfindung betrifft einen Hüllkörper zur zumindest teilweisen Umhüllung einer Gliedmaße mit einem eingeschlossenen Volumen und einem Anschluss zum Zuführen und Abführen von Fluid in das Volumen oder aus dem Volumen, wobei der Hüllkörper einen Innenumfang und einen Außenumfang ausbildet. Der Hüllkörper ist insbesondere zur Anlage an einen Stumpf oder an einer Gliedmaße vorgesehen.

Zwischen einem Prothesenschaft und einem Stumpf einer Gliedmaße ist zur Polsterung des Stumpfes, zum Ausgleichen von Unebenheiten der Schaftoberfläche sowie zum Herstellen einer Verbindung zwischen dem Stumpf und dem Prothesenschaft in der Regel ein Liner vorgesehen, der über den Stumpf gezogen wird. Der Liner haftet an der Hautoberfläche des Stumpfes und kann an seinem distalen Ende Verriegelungselemente aufweisen, beispielsweise einen Pin oder ähnliches, um den Liner mit einer korrespondierenden mechanischen Verriegelungseinrichtung in dem Prothesenschaft festzulegen und zu verhindern, dass der Liner entgegen der Einführrichtung aus dem Prothesenschaft herausbewegt wird. Wird der Prothesenschaft über ein Vakuum an dem Liner festgelegt, wird zwischen dem Liner und dem Prothesenschaft ein abgedichtetes Volumen ausgebildet, aus dem über eine Pumpe oder ein Ventil Luft herausgefördert wird. Über den Unterdruck wird erreicht, dass der Prothesenschaft an dem Liner gehalten wird.

Prothesenliner bestehen in der Regel aus einem Elastomerwerkstoff, beispielweise Silikon, der eine hohe Haftfähigkeit an dem Stumpf aufweist. Zum Anlegen wird der Liner aufgerollt, das distale, vorzugsweise geschlossene Ende des Prothesenliners wird auf das Stumpfende aufgelegt und der Liner dann über den Stumpf abgerollt. Regelmäßig weist der Liner einen leicht geringeren Innendurchmesser als der Außendurchmesser des Stumpfes auf, um einen vergleichsweise festen Sitz an dem Stumpf zu ermöglichen.

Aus der US 5,387,245 A ist ein Prothesenliner mit einem Innenliner und einem Außenliner aus einem Elastomermaterial bekannt. Der Innenliner und der Außenliner sind miteinander verklebt, wobei zur Ausbildung einer Blase zwischen dem Innenliner und dem Außenliner ein Bereich unverklebt ist. Ein Ventil ist der Blase zugeordnet und ermöglicht es, Luft in die Blase einzuführen oder aus ihr abzulassen, um Volumenschwankungen des Stumpfes ausgleichen zu können.

Die US 2013/0218296 A1 betrifft eine Prothese mit einem Schaft und einem Liner, wobei in einem Bereich zwischen dem Schaft und dem Liner ein Fluid eingebracht wird. Die Fluidmenge in dem Bereich kann reguliert werden. Für ein erleichtertes Anlegen der Prothese an einen Amputationsstumpf wird der Innenumfang des Liners geweitet, indem Fluid aus dem Bereich abgepumpt wird. Anschließend wird Fluid in den Bereich zurückgefördert, um den Liner fest an den Amputationsstumpf anzulegen.

Die EP 2 327 378 B1 betrifft eine Kappe für eine Prothese, die an einen Amputationsstumpf angelegt und an einem Prothesenschaft befestigt wird. Die Kappe besteht aus zwei Membranen, die eine luftdichte Kammer ausbilden. In die luftdichte Kammer wird ein Fluid eingebracht, um den Tragekomfort zu verbessern.

Die DE 917 687 B betrifft eine doppelwandige Fassung für einen Stumpf mit einem Hohlraum, der mittels einer Reihe von Geweben in miteinander verbundene Abteilungen unterteilt wird. An der Fassung sind ein Rohr und ein Saugpfropfen angeordnet, über die die Abteilungen des Hohlraumes mittels eines Blasebalgs oder einer anderen Pumpe aufgeblasen werden können.

Die US 2003/0078674 A1 betrifft einen Prothesenschaft mit einem an seiner Innenseite angeordneten System aus Kammern, in die ein Fluid eingebracht wird. Wenn der Prothesenschaft an einem Amputationsstumpf angelegt ist, können die Fluidmengen in den Kammern verändert werden. Eine Veränderung des Stumpfvolumens kann hierdurch ausgeglichen werden, um den Tragekomfort der Prothese zu gewährleisten.

Die US 1 057 562 A beschreibt eine Stumpfabstützung bestehend aus einem rechteckigen aufblasbaren Kissen mit einer größeren Länge als der Umfang des Beines, so dass das aufblasbare Kissen überlappend um den Stumpf herum gelegt werden kann. Das Kissen erstreckt sich über und stützt sich auf einer oberen Kante eines Prothesenschaftes ab und ist im Bereich der oberen Kante mit einem Ventil ausgestattet.

Die US 5,108,456 A betrifft eine Prothese mit einer harten Außenschale und einem darin angeordneten Schaft, wobei an dem Schaft eine Vielzahl aufblasbarer Luftkammern angeordnet ist. Die Luftkammern können aufgeblasen und entleert werden, um den Tragekomfort und die Stabilität der an einem Amputationsstumpf angelegten Prothese zu verbessern.

Die SU 731 963 betrifft eine Prothese für eine untere Gliedmaße. In einem starren Prothesenschaft sind aufblasbare Schläuche zickzackartig angeordnet, um eine Anpassung an den Stumpf zu erleichtern.

Weiterhin existieren aus dem Stand der Technik Bandagen oder Manschetten, die um eine Gliedmaße herum angelegt werden, um einen umfänglichen Druck auf die Gliedmaße auszuüben. Solche Bandagen oder Manschetten sind bevorzugt aus einem Gewebe oder einem Schaumwerkstoff hergestellt. Zum Anlegen müssen diese Hüllkörper, die sowohl eine proximale als auch eine distale Öffnung aufweisen, ebenfalls gedehnt werden.

Die DE 10 2016 101 670 A1 betrifft einen Hüllkörper zur zumindest teilweisen Umhüllung einer Gliedmaße mit einem eingeschlossenen Volumen und einem Anschluss zum Zuführen und Abführen von Fluid in das Volumen oder aus dem Volumen. Der Hüllkörper bildet einen Innenumfang und einen Außenumfang aus, wobei sich der Innenumfang des Hüllkörpers bei einer Druckerhöhung des Volumens vergrößert, um das Anlegen des Hüllkörpers zu erleichtern.

Die US 2016/0270479 A1 betrifft einen Schuh, der leicht anzuziehen und leicht auszuziehen sein soll. Er soll billig, leicht, komfortabel, gesundheitsfördernd und längeneinstellbar sein. Dies wird dadurch erreicht, dass die Formen einer äußeren Schicht und innenliegender Volumina korrespondierend zu verschiedenen Körperteilen angeordnet sind. Sowohl die äußere Schicht als auch die Volumina bestehen aus einem dehnbaren Material, die hauptsächlich mit der Innenseite der äußeren Schicht verbunden sind. Über Ventile können die Volumina mit Luft befüllt oder entleert werden. Wird Luft in die Volumina eingeleitet, dehnt sich der gesamte Schuh aufgrund der Flexibilität aus und erleichtert das Anziehen des Schuhs. Nachdem der Fuß eingeführt wurde, werden die Ventile geöffnet und Luft wird durch das Zusammenziehen der Volumina herausgedrückt. Die Volumina zusammen mit der äußeren Schicht des Schuhs treten in einen engen Kontakt mit dem Fuß, so dass der Schuh ohne Rutschen getragen werden kann.

Die WO 2013/113332 A1 wird als nächstliegender Stand der Technik angesehen und betrifft eine reversibel versteifbare Retentionsvorrichtung mit einer flexiblen Hülle mit einer Außenseite und einem Innenraum sowie ein in dem Innenraum der Hülle angeordnetes, schichtförmiges Füllmaterial, das wenigstens zwei übereinandergestapelten Schichten aufweist. Die Schichten bestehen aus einer Vielzahl von gegeneinander beweglichen Teilflächen, wobei jede Teilfläche mit mindestens einer benachbarten Teilfläche innerhalb einer Schicht verbunden ist. Zum Versteifen der Retentionsvorrichtung wird in dem Innenraum ein Druck unterhalb des atmosphärischen Druckes erzeugt.

Vorteilhaft an den Lösungen aus dem Stand der Technik ist die hohe Flexibilität und Elastizität der Hüllkörper, wie Manschetten oder Liner. Nachteilig ist die Notwendigkeit eines starren Prothesenschaftes oder Rahmens, um weitere Komponenten an einer Gliedmaße festlegen zu können.

Aufgabe der vorliegenden Erfindung ist es daher, einen Hüllkörper bereitzustellen, der einerseits eine individuelle Anpassung an den jeweiligen Stumpf ermöglicht und andererseits eine einfache Ankopplung weiterer Komponenten erleichtert. Erfindungsgemäß wird diese Aufgabe durch einen Hüllkörper mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Der erfindungsgemäße Hüllkörper zur zumindest teilweisen Umhüllung einer Gliedmaße mit einem eingeschlossenen Volumen und einem Anschluss zum Zuführen und Abführen von Fluid in das Volumen oder aus dem Volumen, wobei der Hüllkörper einen Innenumfang und einen Außenumfang ausbildet, sieht vor, dass an dem Hüllkörper Versteifungselemente angeordnet sind, die ausgebildet sind durch ein Abführen von Fluid aus dem Volumen in Kontakt gebracht zu werden oder ihren Kontakt miteinander durch ein Abführen von Fluid zu verstärken. Durch das Inkontaktbringen der Versteifungselemente miteinander oder das Verstärken des Kontaktes der Versteifungselemente miteinander nach dem Ablassen oder Evakuieren, z.B. durch Abpumpen, von Fluid aus dem Volumen ist es möglich, den Liner an die aktuelle Stumpfform bei jedem Anziehvorgang und nach Bedarf anpassen zu können und gleichzeitig darüber eine versteifte, ggf. rigide Form des Hüllkörpers bereitzustellen, so dass an den Hüllkörper im angelegten Zustand nach Inkontaktbringen der Versteifungselemente oder der Verstärkung des Kontaktes oder der Anpresskräfte aneinander eine ausreichende Steifigkeit vorhanden ist, so dass weitere Komponenten, wie Orthesen- oder Prothesengelenke an den Hüllkörper angelegt und daran befestigt werden können. Mit dem Hüllkörper ist es möglich, das variable Verhalten eines elastischen Liners mit dem rigiden Verhalten eines Prothesenschaftes zu kombinieren, so dass mit einem standardmäßig ausgebildeten Hüllkörper eine individuelle Anpassung an den jeweiligen Nutzer bei einem nicht evakuierten oder drucklosen Zustand des Hüllkörpers oder mit Überdruck versehenen Volumen erreicht werden kann. Bei einem befüllten oder drucklosen Zustand des Volumens kann der Hüllkörper aufgeweitet oder flexibel an die Gliedmaße angepasst werden. Wird das Fluid abgelassen, legt sich der Hüllkörper an die Gliedmaße an und die Versteifungselemente werden miteinander in Kontakt gebracht oder bei einem bereits bestehenden Kontakt werden die Anpresskräfte erhöht. Wird aktiv ein Unterdruck gegenüber der Umgebung in dem Volumen erzeugt, wird eine erhöhte Anpressung und Versteifung des Hüllkörpers erreicht. Nach der Evakuierung bildet sich eine stabilere, ggf. versteifte oder rigide Struktur aus, so dass der Hüllkörper als Prothesenschaft oder formstabile Abstützung der umschlossenen Gliedmaße dienen kann. Der Liner kann nach Adaption auf das Volumen der umschlossenen Gliedmaße oder des Stumpfes reversibel zu einer starren Umhüllung oder einem Schaft ausgebildet werden.

In einer Ausgestaltung der Erfindung sind die Versteifungselemente innerhalb des Volumens angeordnet, wodurch sich eine glatte Innen- und Außenkontur bei gleichzeitigem Schutz der Versteifungselemente vor äußeren Einflüssen erreichen lässt. Alternativ oder ergänzend sind die Versteifungselemente an dem Außenumfang des Hüllkörpers angeordnet und werden durch das Abführen von Fluid aus dem Volumen in Kontakt miteinander gebracht oder in ihrem Kontakt verstärkt, wenn sich der Außenumfang durch das Ablassen des Fluids verringert.

Der Hüllkörper kann zumindest zwei Wände aufweisen, zwischen denen das Volumen eingeschlossen ist, wobei die Versteifungselemente beispielsweise auf der dem Volumen zugewandten Seite angeordnet und/oder in diese integriert sind. Dadurch ist es möglich, dass die jeweiligen Außenseiten des Hüllkörpers unbeeinflusst von der Formgebung der Versteifungselemente ausgebildet werden können, die Innenseite des Hüllkörpers ist bevorzugt glattwandig ausgebildet, um eine gleichmäßige Anlage an die Gliedmaße oder den Stumpf zu ermöglichen. An der Außenseite des Hüllkörpers können Befestigungselemente oder Aufnahmen für weitere Komponenten angeordnet sein, um den Hüllkörper mit weiteren Komponenten zu verbinden. Bevorzugt sind die Versteifungselemente auf einander gegenüberliegenden, einander zugewandten Seiten der beiden Wände oder als separate Lage zwischen den Wänden angeordnet und werden durch Abführen von Fluid oder Evakuieren des Volumens in Kontakt miteinander oder mit der gegenüberliegenden Wand gebracht, um den Hüllkörper zu versteifen. Sind die Versteifungselemente in den Wänden oder in zumindest einer Wand integriert, kann eine kompakte Bauweise erreicht werden.

Eine Weiterbildung der Erfindung sieht vor, dass in dem Volumen einander gegenüberliegende oder nebeneinander angeordnete Versteifungselemente formschlüssig miteinander in Eingriff bringbar sind. Die Versteifungselemente müssen dabei nicht an einander gegenüberliegenden Innenwänden des Volumens angeordnet sein, es besteht auch die Möglichkeit, dass die Versteifungselemente geschuppt übereinander oder nebeneinander angeordnet sind und dadurch im nicht evakuierten Zustand des Hüllkörpers beweglich zueinander an dem Hüllkörper an nur einer Wand befestigt sind. Erst wenn die Versteifungselemente miteinander in Kontakt gebracht werden, entweder auf einander gegenüberliegenden Seiten oder durch Anpressen einer Wand des Volumens an die innerhalb des Volumens befindlichen Versteifungselemente oder durch aneinander Anlegen oder in Eingriff bringen durch eine Umfangsverringerung bei nebeneinander angeordneten Versteifungselementen, wird die Beweglichkeit der Versteifungselemente zueinander behindert oder verhindert und eine Versteifung des gesamten Hüllkörpers zumindest in dem Bereich der Versteifungselemente erreicht. Die Versteifungselemente verriegeln sich miteinander und verhindern dadurch eine Relativbewegung zueinander. Die Versteifungselemente sind beispielsweise an einer Wand befestigt, beispielsweise stoffschlüssig oder formschlüssig und übertragen Kräfte und Momente von der Gliedmaße auf den Außenumfang und/oder umgekehrt. Die Versteifungselemente sind bevorzugt starre, formstabile Komponenten oder Elemente, die Kräfte und Momente weiterleiten, ohne dass die sich verformen oder eine wesentliche Verformung zulassen, um so in Verbindung mit anderen Versteifungselementen, die miteinander in Kontakt gebracht werden oder deren Anpresskräfte verstärkt werden, eine Versteifung des Hüllkörpers zu bewirken. Die Versteifungselemente können auch über andere Mechanismen mit den Wänden kraftübertragend oder momentenübertragend verbunden sein, beispielsweise durch Reibung oder Anpressen über Unterdruck.

In einer Variante ist der Anschluss als ein Sauganschluss mit einer Sperreinrichtung ausgebildet ist, die nach einem Evakuieren des Volumens und einem Aneinanderpressen der Versteifungselemente ein Rückströmen von Fluid in das Volumen verhindert. Ein Öffnen des Anschlusses kann gegebenenfalls nicht ausreichen, um einen ausreichenden Anpressdruck der Versteifungselemente aneinander zu erzeugen oder die Versteifungselemente in einen ausreichenden Kontakt miteinander zu bringen. Zwar verkleinert sich durch das Ablassen des Fluids das Volumen und damit auch der Außenumfang des Hüllkörpers, es besteht jedoch die Möglichkeit, dass für den gewünschten Einsatzzweck die dadurch erreichte Verfestigung, Stabilisierung oder Versteifung nicht ausreicht. Daher ist der Anschluss in einer Variante als ein Sauganschluss ausgebildet, der mit einer Saugeinrichtung, beispielsweise einer Vakuumpumpe, in strömungstechnische Verbindung gebracht werden kann, um weiteres Fluid aus dem Volumen abzusaugen und einen Innendruck innerhalb des Volumens herzustellen, der unterhalb des Umgebungsdruckes liegt. Je größer die Druckdifferenz zwischen dem Volumen und der Umgebung eingestellt wird, desto stärker werden die Versteifungselemente miteinander in Kontakt gebracht oder ihr Konktakt miteinander verstärkt, sodass sich der Versteifungseffekt vergrößert. Die an der Gliedmaße angenommene Form des Hüllkörpers wird durch die Evakuierung festgelegt und eine Versteifung herbeigeführt.

Die Versteifungselemente können an ihren Kontaktflächen einander korrespondierende Oberflächenstrukturen aufweisen, beispielsweise Noppen, Stege, Blöcke, Vorsprünge und Hinterschneidungen, Hakenstrukturen, parallel ausgerichtete Borsten oder Streben, Strukturgewebe, strukturierte Vliesstoffe, Gewebe oder Gewirke mit einer gemeinsam ausgerichteten Strichorientierung. Werden diese Oberflächenstrukturen aneinandergelegt, versteift sich der Hüllkörper zumindest bereichsweise. Alternativ oder ergänzend weisen die Versteifungselemente zumindest an ihren Kontaktflächen einen Reibungskoeffizienten auf, der größer oder gleich 0,3 ist. Der Reibungskoeffizient wird möglichst groß gewählt, auch wenn die Haft- und Haltebedingungen zusätzlich von der Geometrie der Versteifungselemente, der Flächen und des Anpressdruckes beeinflusst werden. Bevorzugt wird eine Werkstoffpaarung der aneinander anliegenden oder in Kontakt zu bringenden Versteifungselemente gewählt, die eine Relativverlagerung zueinander möglichst erschwert. Die Versteifungselemente können aus unterschiedlichen Materialien ausgebildet oder mit Bereichen mit unterschiedlichen Materialien versehen sein, sodass eine Werkstoffpaarung an den Kontaktflächen bereitgestellt wird, die einen möglichst hohen Reibungskoeffizienten aufweisen, um eine Relativverlagerung der Versteifungselemente bei Kontakt zu vermeiden oder zumindest zu erschweren.

Die nicht in Kontakt miteinander befindlichen Versteifungselemente sind bevorzugt verlagerbar zueinander an oder in dem Hüllkörper angeordnet. Besteht das Versteifungselement aus mehreren Komponenten, die an der gleichen Wand befestigt sind, werden diese miteinander in Kontakt gebracht und versteifen den Hüllkörper. Gleiches gilt für ebenso ausgebildete Versteifungselemente auf einander gegenüberliegenden Wänden, so dass die modular aufgebauten Versteifungselemente an beiden Enden bei Evakuierung des Volumens durch Aneinanderanlegen oder Miteinanderverriegeln in Kontakt gebracht werden. Dabei kann eine Kombination aus Versteifungseffekten durch Aneinanderlegen und Inkontaktbringen von Komponenten eines Versteifungselementes mit dem Aneinanderlegen und Inkontaktbringen einander gegenüberliegender Versteifungselemente erfolgen.

Eine Weiterbildung der Erfindung sieht vor, dass der Hüllkörper eine Innenwand und eine Außenwand aufweist, die das zumindest eine Volumen einschließen.

Das zumindest eine Volumen kann miteinander verbundene Bereiche aufweisen und/oder sich wendeiförmig in proximal-distal Richtung des Hüllkörpers erstrecken. Dazu kann das zumindest eine Volumen durch zumindest eine Zwischenwand in unterschiedliche, aber miteinander verbundene Bereiche unterteilt sein, die beispielsweise durch Strömungskanäle oder Ventile miteinander verbunden sind. Durch die Ausgestaltung mit Ventilen ist es möglich, unterschiedliche Druckniveaus innerhalb des Volumens einzustellen. Die Bereiche erstrecken sich bevorzugt über die gesamte Länge des Hüllkörpers, also von einem proximalen Ende bis zu einem distalen Ende, wobei das Volumen oder die Bereiche nach Art einer Wendelfeder oder eines Gewindes ausgebildet sein können.

Darüber hinaus besteht die Möglichkeit, dass der Hüllkörper mehrere über den Umfang verteilte, durch zumindest eine Zwischenwand getrennte Volumina aufweist, so dass einzelne, um den Umfang verteilte Kammern ausgebildet werden, die es ermöglichen, eine unterschiedliche Evakuierung durchzuführen oder auch einzelne Kammern gar nicht zu evakuieren. Alternativ oder ergänzend können Kammern radial hintereinander angeordnet sein, also beispielsweise ringförmig oder ringabschnittsförmig um den Umfang des Stumpfes oder der Gliedmaße angeordnet sein, insbesondere wenn der Hüllkörper als geschlossener Querschnitt mit einer tubusartigen Kontur ausgebildet ist. Dann werden zwei Kammern durch drei beispielsweise umfänglich durchgehende Wände ausgebildet. Diese Ringwände bilden zwischen einander Ringspalte aus, die zwei Volumina ausbilden, die mit Fluid befüllt oder evakuiert werden können. Durch weitere Ringwände ist es möglich, weitere Volumina hinzuzufügen. Die einzelnen Ringspalte oder Ringvolumina können durch Zwischenwände oder Unterteilungen abgetrennt werden, so dass eine Vielzahl um den Umfang verteilter des Hüllkörpers verlaufender Kammern in unterschiedlicher Dicke und unterschiedlicher Breite ausgebildet werden können.

Die Innenwand und/oder die Zwischenwand und/oder die Außenwand sind faltbar und/oder elastisch ausgebildet, um eine einfache Verformung, Aufweitung und Evakuierung zu ermöglichen. Um einen möglichst individuell anpassbaren Hüllkörper zu realisieren, ist in einer Weiterbildung der Erfindung jedem Volumen ein Anschluss zum Zuführen und Abführen von Fluid zugeordnet. Die einzelnen Volumina können über Ventile, beispielsweise einstellbare Ventile, miteinander verbunden sein und Fluidströmungen durch einzelne Kammern in andere Kammern ermöglichen.

In einer Weiterbildung der Erfindung ist vorgesehen, dass die Versteifungselemente als Streben, Platten, Granulat, Filamente, Gewebe, Gewirke, Gestricke und/oder strukturiertes Flächenmaterial ausgebildet sind.

Der Innenumfang des Hüllkörpers kann sich bei einer Druckerhöhung des Volumens vergrößern, um das Anlegen eines Hüllkörpers zu erleichtern, insbesondere wenn der Innenumfang als geschlossener Innenumfang ausgebildet und der Hüllkörper als Liner oder Manschette ausgebildet ist. Dadurch wird das Anlegen erleichtert, da der Innenumfang vergrößert wird und die Gliedmaße oder der Stumpf leicht in einen vergrößerten Innenhohlraum eingeführt werden kann. Nach Ablassen von Fluid aus dem Volumen oder den Volumina legt sich der Innenumfang des Hüllkörpers an den Stumpf oder die Gliedmaße an und passt sich aufgrund der flexiblen und vorzugsweise elastischen Ausgestaltung der Innenwand an die jeweilige Kontur an.

Der Hüllkörper kann eine zumindest teilweise haftende Innenoberfläche aufweisen, um nach dem Anlegen des Hüllkörpers eine Relativbewegung zu der Hautoberfläche zu vermeiden oder zu vermindern.

Der Hüllkörper kann als Prothesenliner, Bandage, Manschette, Kleidungsstücke oder Schuhe ausgebildet sein. Bei einer Ausgestaltung des Hüllkörpers als Prothesenliner ist vorzugsweise eine Befestigungseinrichtung für eine festzulegende weitere Prothesenkomponente an dem Hüllkörper angeordnet oder ausgebildet.

Um bei einer Vergrößerung der eingeschlossenen Volumina eine Vergrößerung des Innenhohlraumes eines Hüllkörpers zu erleichtern, sind in oder an dem Hüllkörper zugkraftübertragende Elemente angeordnet, die zugstarr oder elastisch ausgebildet sein können. Die zugkraftübertragenden Elemente sind bevorzugt zwischen zwei Wänden, z.B. zwischen einer Innen- und Außenwand oder zwischen einer Zwischenwand und einer Innen- und/oder Außenwand, des Hüllkörpers angeordnet und befinden sich innerhalb eines von den Wänden eingeschlossenen Volumens. So wird bei einem Ausdehnen des Hüllkörpers aufgrund einer Druckerhöhung das Volumen für die aufzunehmende Gliedmaße oder den aufzunehmenden Stumpf vergrößert.

Nachfolgend werden Ausführungsbeispiele der Erfindung anhand der beigefügten Figuren näher erläutert. Es zeigen:
- Figur 1 -: eine schematische Darstellung eines Hüllköpers in Gestalt eines Prothesenliners in zwei Zuständen;
- Figur 2 -: eine Darstellung eines Hüllkörpers als Prothesenliner in perspektivischer Ansicht;
- Figur 3 -: eine schematische Darstellung einer Bandage;
- Figur 4 -: eine schematische Darstellung eines Hüllkörpers in Gestalt eines Schuhs;
- Figur 5 -: eine Schnittdarstellung durch einen Liner mit Versteifungselementen;
- Figur 6 -: eine Variante des Liners mit einer Zwischenwand; sowie
- Figur 7 -: eine Variante der Erfindung mit einer Armierung.

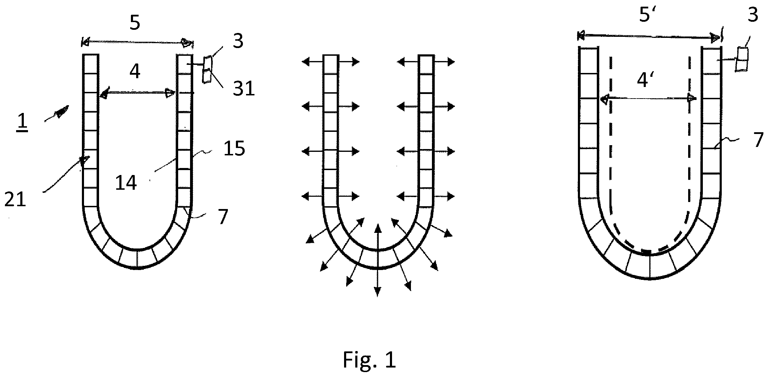

Figur 1 zeigt eine schematische Darstellung eines orthopädietechnischen Hüllkörpers 1 in Gestalt eines Prothesenliners als grundsätzliche Wirkungsweise der Erfindung. Der Hüllkörper 1 weist eine Innenwand 14 und eine Außenwand 15 auf, die zwischen sich ein Volumen 21 einschließen. Der Hüllkörper 1 ist somit doppelwandig ausgebildet und weist einen Anschluss 3 mit einer Sperreinrichtung 31 in Gestalt eines Ventils auf, um das Volumen 21 zwischen der Innenwand 14 und der Außenwand 15 mit einem Fluid zu befüllen oder Fluid aus dem Volumen 21 abzulassen. Das Fluid ist bevorzugt Umgebungsluft. Zwischen der Innenwand 14 und der Außenwand 15 sind eine Vielzahl zugkraftübertragender Elemente 7 ausgebildet, die im dargestellten Ausführungsbeispiel als Stege ausgebildet sind. Es besteht auch die Möglichkeit, statt der Stege Gurte, Stifte oder auch nur Verbindungspunkte zwischen der Innenwand 14 und der Außenwand 15 vorzusehen. Im nicht mit Fluid befüllten Zustand kann die Innenwand 14 an der Außenwand 15 anliegen.

Der Hüllkörper 1 ist im Längsschnitt U-förmig ausgebildet, im Querschnitt senkrecht zu der Längserstreckung des Hüllkörpers 1 weist dieser eine im Wesentlichen kreisförmige, geschlossene Kontur auf. Im dargestellten, nicht befüllten Zustand ist somit ein Innenumfang 4 und ein Außenumfang 5 vorhanden, die Abmessungen der Umfänge 4, 5 ergeben sich aus der Formgebung, der Dimensionierung und den Materialeigenschaften.

Wird durch den Anschluss 3 Fluid in das abgeschlossene Volumen 21 eingeleitet, erhöht sich der Druck innerhalb des Volumens 21, da sowohl die Innenwand 14 als auch die Außenwand 15 aus einem elastischen Material hergestellt sind, vorzugsweise aus einem Elastomer oder einer Verbindung aus elastischen Abschnitten und unelastischen Abschnitten. Durch den erhöhten Innendruck wirken entsprechende Kräfte auf die Innenwand 14 und die Außenwand 15, wobei aufgrund der größeren Fläche der Außenwand 15 dort größere Kräfte wirken. Bei gleichen Materialeigenschaften hinsichtlich der Dehnung, beispielsweise wenn die Innenwand 14 und die Außenwand 15 aus dem gleichen Material bestehen, ergibt sich hieraus, dass aufgrund der größeren Kräfte auf die Außenwand 15 eine größere Verformung und Dehnung stattfindet, was dazu führt, dass insgesamt der Hüllkörper 1 ausgedehnt wird. Dies ist in der rechten Darstellung gezeigt. Der Außenumfang 5'im druckbeaufschlagten Zustand ist im Vergleich zur linken Darstellung vergrößert. Auch der Innenumfang 4' ist im Vergleich zur Ausgangsstellung, die in der Strichlinie dargestellt ist, vergrößert, ebenso wie der Abstand zwischen der Innenwand 14 und der Außenwand 15.

Durch die Druckbeaufschlagung über das Ventil 31 und den Anschluss 3 ist es möglich, den Innendurchmesser und damit auch den Umfang 4 des Aufnahmeraums des Hüllkörpers 1 für die Gliedmaße oder den Stumpf zu vergrößern, so dass der nicht dargestellte Stumpf leicht eingeführt werden kann. Wird das Ventil 31 an dem Zugang 3 geöffnet, entweicht Luft aus dem Volumen 21 aufgrund der elastischen Rückstellkräfte, die durch die Innenwand 14 und die Außenwand 15 bereitgestellt werden. Das Volumen des Aufnahmeraumes für den Stumpf verkleinert sich, die Innenwand 14 legt sich an den nicht dargestellten Stumpf an und es wird ein fester Sitz des Hüllkörpers 1 an dem Stumpf ermöglicht. Je nach Grad der Druckminderung ergibt sich eine angepasste Anpresskraft der Innenwand 14 an den Stumpf, wobei die maximale Anpresskraft dann erreicht ist, wenn das Volumen 21 minimiert wird.

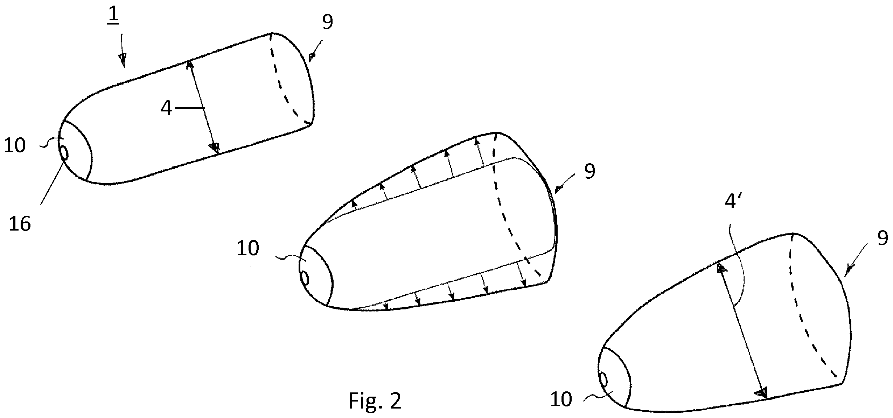

Figur 2 zeigt eine Ausführungsform des Hüllkörpers 1 in Gestalt eines Prothesenliners mit einer proximalen Einführöffnung 9 und einem geschlossenen distalen Endbereich 10. An dem distalen Endbereich 10 können eine formstabile Kappe sowie eine Befestigungseinrichtung 16 wie ein Pyramidenadapter für eine mechanische Verriegelung mit anderen Prothesenkomponenten wie Kniegelenken oder dergleichen angeordnet sein. Der Prothesenliner 1 weist in der oberen Darstellung einen ersten Innenumfang 4 auf. Von dem distalen Endbereich 10 erstreckt sich eine Seitenwand im Wesentlichen konisch in Richtung auf die proximale Einstiegsöffnung 9. Die Seitenwand oder der Seitenwandbereich ist doppelwandig ausgebildet, der distale Endbereich 10 ist entweder einlagig ausgebildet oder die beiden Schichten eines doppelwandigen Prothesenliners sind miteinander verklebt oder stoffschlüssig miteinander verbunden. In dem distalen Endbereich 10 kann kein Druckfluid eingeleitet werden.

Die mittlere Darstellung der Figur 2 zeigt die wirksamen Kräfte beim Einleiten eines Druckfluids in den in der Seitenwand ausgebildeten Hohlraum oder in das Volumen, das zwischen einem Innenliner und einem Außenliner eingeschlossen ist. In der rechten Darstellung ist die Form des Prothesenliners 1 bei einem erhöhten Volumeninnendruck dargestellt. Der Innenumfang 4'ist größer als der Ausgangsinnenumfang 4, die Einstiegsöffnung 9 des im distalen Endbereich 10 geschlossenen Prothesenliners ist im Vergleich zur ursprünglichen Einstiegsöffnung 9 wesentlich vergrößert. Dadurch wird das Einsteigen in den Prothesenliner 1 erleichtert. Nachdem das Stumpfende Kontakt mit der Innenseite des distalen Endbereiches 10 aufgenommen hat, wird das Ventil 31 an dem Anschluss 3 geöffnet, die Luft entweicht und der Prothesenliner 1 legt sich mit der Seitenwand an den Stumpf an.

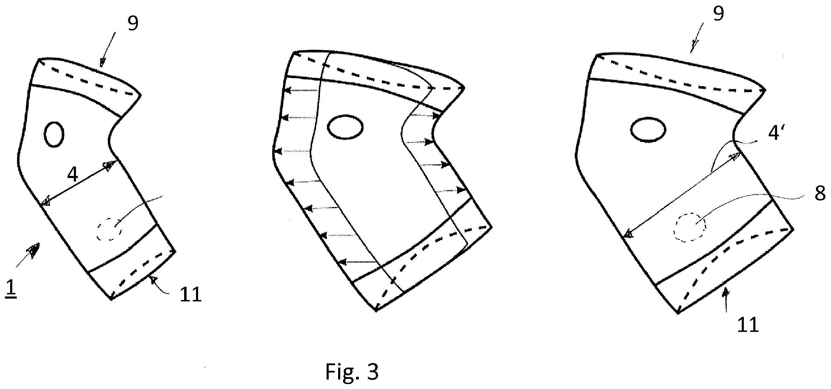

Eine alternative Ausführungsform der Erfindung ist in Figur 3 dargestellt, bei der der Hüllkörper 1 in Gestalt einer pneumatisch veränderlichen Bandage ausgestaltet ist. Die Bandage kann als Kniebandage ausgebildet sein und ein Gelenk überbrücken. Alternativ ist die Ausgestaltung als Ellenbogenbandage, Handgelenksbandage oder Knöchelbandage möglich. Darüber hinaus kann die Bandage auch nicht gelenkübergreifend ausgebildet sein und beispielsweise den Oberschenkel, Oberarm, Unterschenkel oder Unterarm vollumfänglich umgreifen. Der Querschnitt der Bandage ist im dargestellten Ausführungsbeispiel geschlossen. Über die gesamte Länge der Bandage 1, die sich zwischen der proximalen Einstiegsöffnung 9 und der distalen Durchstiegsöffnung 11 erstreckt, ist sie doppelwandig ausgebildet und kann über einen nicht dargestellten Anschluss mit Druckluft beaufschlagt werden. Die Druckluftbeaufschlagung ist in der mittleren Darstellung der Figur 3 gezeigt, in der angedeutet ist, dass aus dem in der linken Darstellung gezeigten Ausgangszustand die Bandage 1 in den in der rechten Darstellung gezeigten Endzustand überführt wird. Der Innenumfang 4' in dem druckbeaufschlagten Zustand gemäß der rechten Darstellung ist größer als der Ausgangsinnenumfang 4, so dass die Bandage einfach angelegt werden kann. Eine Kompression findet durch Ablassen von Druckluft statt. An der Innenoberfläche der Bandage 1 können Bereiche 8 mit einer erhöhten Haftfähigkeit ausgebildet sein, so dass der Hüllköper 1 auf seiner Innenoberfläche 8 zumindest teilweise eine haftende Beschichtung aufweist.

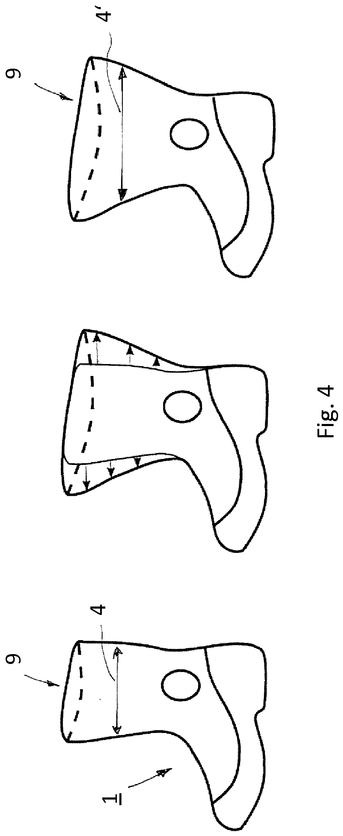

Figur 4 zeigt eine weitere Variante der Erfindung, bei der der Hüllkörper 1 als pneumatisch betriebener Schuh oder Stiefel ausgebildet ist. Der Schaftbereich des Hüllkörpers 1, der sich oberhalb des natürlichen Knöchelgelenks erstreckt, ist doppelwandig ausgebildet und bildet ein hülsenartiges Volumen aus, das mit Druckluft befüllt werden kann. Der Befüllvorgang ist in der mittleren Darstellung gezeigt, in der rechten Darstellung ist zu erkennen, dass die proximale Einstiegsöffnung 9 im Vergleich zur Ausgangsausdehnung wesentlich vergrößert ist, ebenso ist der Innenumfang 4'vergrößert zum ursprünglichen Innenumfang 4, so dass das Einsteigen in den Schuh oder Stiefel 1 wesentlich erleichtert wird. Nach dem Einsteigen kann der Druck in dem Volumen 21 reduziert werden, wodurch sich der Schaft an den Unterschenkel anlegt.

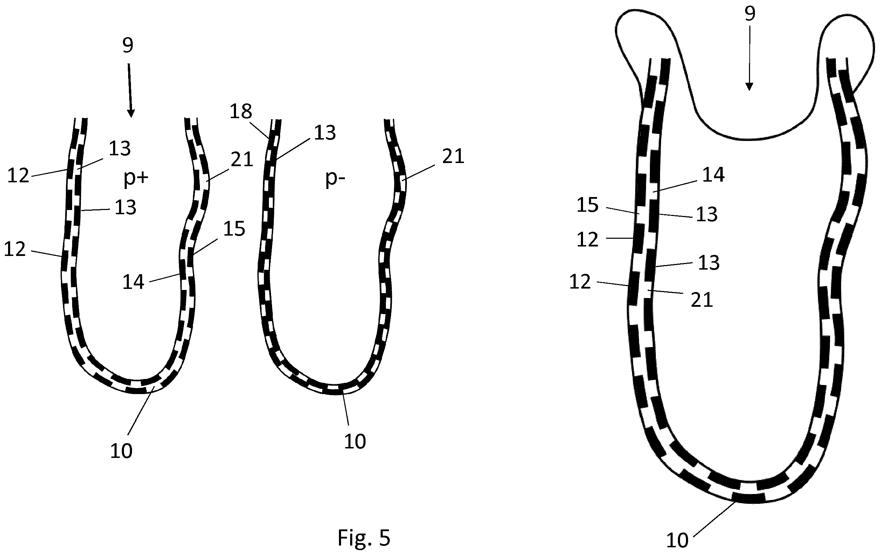

Die Figur 5 zeigt in der linken Darstellung eine Teilschnittansicht eines Hüllkörpers 1 in Gestalt eines Prothesenliners mit einem befüllten Volumen 21, was durch das p+-Zeichen angedeutet ist, die mittlere Darstellung in Figur 5 zeigt den Ausschnitt in einem evakuierten Zustand, bei dem das Volumen 21 von dem darin befindlichen Fluid befreit wurde. Dies ist durch das Zeichen P- verdeutlicht. Innerhalb des Volumens 21, das zwischen der Innenwand 14 und der Außenwand 15 ausgebildet ist, sind Versteifungselemente 12 angeordnet, die innerhalb des Volumens 21 einander gegenüberliegen. In der linken Darstellung der Teilschnittdarstellung sind die Versteifungselemente 12, 13 voneinander beabstandet, da sich ein Fluid, beispielsweise Luft, innerhalb des Volumens 21 befindet und beispielsweise einen Überdruck gegenüber der Umgebung ausbildet. In dem dargestellten Ausführungsbeispiel sind die Versteifungselemente 12, 13 als separate Komponenten ausgebildet, beispielsweise als Einzelplatten auf der Innenseite der Außenwand 15 und der dem Volumen zugewandten Seite der Innenwand 14. Wird nun das Fluid innerhalb des Volumens 21 abgelassen, beispielsweise durch den nicht dargestellten Anschluss 3, an den eine Pumpe angeschlossen sein kann, adaptiert die Innenwand 14 die individuelle Form des umschlossenen oder eingeschlossenen Stumpfes. Anschließend oder gleichzeitig legen sich die beiden Wände 14, 15 aneinander an und bringen die Versteifungselemente 12, 13 miteinander in Kontakt oder verstärken den Kontakt. Wird dann ein Unterdruck angelegt, werden die Innenwand 14 und die Außenwand 15 mit den daran oder darin befindlichen Versteifungselementen 11, 12 aneinander gepresst. Durch das Verpressen oder verstärktes Inkontaktbringen der Versteifungselemente 12, 13, die auf einander gegenüberliegenden Seiten der jeweiligen Wand 14, 15 angeordnet sind, die das Volumen 21 einschließen, versteift sich der Hüllkörper 1 und bildet eine formstabile Umhüllung für den Stumpf aus. Alternativ zu einer Pumpe kann das Fluid aus dem Volumen 21 auch aufgrund elastischer Rückstellkräfte aus dem Volumen 21 herausgedrückt werden, beispielsweise durch Spanneinrichtungen, die zwischen der Innenwand 14 und der Außenwand 15 angeordnet sind und gegen eine Bewegung der Innenwand 14 von der Außenwand 15 weg wirken. Das Ventil oder die Sperreinrichtung 31 verhindert nach dem Evakuieren des Volumens 21 oder der Volumina ein Rückströmen von Fluid, insbesondere Umgebungsluft, so dass die starre Form des Hüllkörpers solange erhalten bleibt, bis das Ventil 31 geöffnet wird und sich die Versteifungselemente 12, 13 relativ zueinander verlagern lassen.

Die Versteifungselemente 12, 13 auf der jeweiligen Wand können entweder als beweglich zueinander ausgebildete Einzelplatten, Noppen, schuppenartige Platten oder andere Komponenten ausgebildet sein, die im entleerten Zustand des Volumens 21 miteinander in Kontakt treten oder in Eingriff treten und eine Verlagerung der Einzelkomponenten zueinander behindern oder verhindern. Die Versteifungselemente 12, 13 können auch einteilig ausgebildet sein, ebenso ist es möglich, dass die Versteifungselemente 12 nur an einer Wand angeordnet sind, wenn die Stabilität der ineinander in Kontakt gebrachten Versteifungselemente 12, 13 ausreicht. Die Versteifungselemente 12, 13 können auch im befüllten Zustand des Volumens 21 aneinander anliegen, sind dann jedoch zueinander beweglich und werden durch Evakuierung und Anlage der jeweils anderen Wand an die Versteifungselemente 12, 13 aufeinander gepresst und in Kontakt gebracht, so dass eine Verlagerung der einzelnen Komponenten der Versteifungselemente 12, 13 zueinander verhindert oder behindert wird.

In der rechten Darstellung der Figur 5 ist ein vollständiger Prothesenliner in einer Teilschnittdarstellung mit den Versteifungselementen 12, 13 im befüllten Zustand des Volumens 21 dargestellt. Die Versteifungselemente 12, 13 können auch als auxetische Flächen ausgebildet sein.

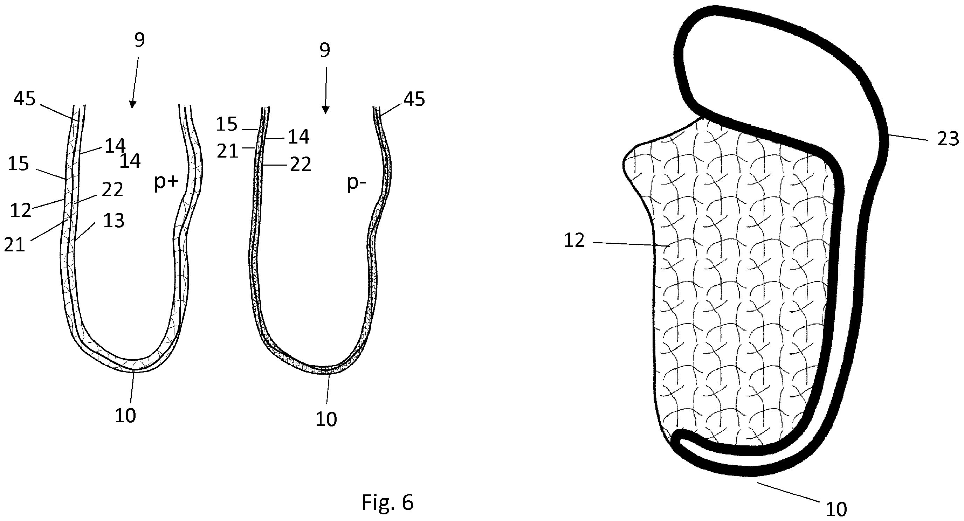

Eine Variante der Erfindung ist in der Figur 6 dargestellt, bei der zwischen der Außenwand 15 und der Innenwand 14 eine Zwischenwand 45 angeordnet ist, so dass sich zwei Volumina 21, 22 in dem Hüllkörper 1 ausbilden. Die geschlossenen Volumina 21, 22 können durch einen separaten Anschluss 3 separat befüllt oder entleert werden. Innerhalb der Volumina 21, 22 sind als Versteifungselemente 12, 13 Filzlagen oder Faserlagen mit gerichteter Orientierung angeordnet, die im befüllten Zustand der Volumina 21, 22, wie er in der ganz linken Darstellung gezeigt ist, eine Verlagerung der Wände 14, 15, 45 zueinander zulassen und aufgrund der Beweglichkeit der Wände 14, 15, 45 ein einfaches Einsteigen und Anlegen des Hüllkörpers 1 in Gestalt eines Prothesenliners ermöglichen. Wird dann das Fluid aus den Volumina 21, 22 abgesaugt, wie es in der mittleren Darstellung gezeigt ist, legen sich die mit Textilelementen belegten Flächen der Wände 14, 15, 45 aneinander an und verriegeln sich miteinander, so dass die Steifigkeit des gesamten Hüllkörpers vergrößert wird. Alternativ zu einer Anordnung von textilen Elementen, wie Filz, Strukturtextilien oder ähnlichen ist es möglich, noppenartige Folien, geriffelte Oberflächen oder andere Oberflächenstrukturen, die eine Verriegelung bewirken, in die Volumina 21, 22 einzubringen und durch Evakuierung oder durch Ablassen des Innendruckes aufeinander zu zubewegen und die Steifigkeit durch Aneinanderanlegen zu erhöhen. An dem distalen Ende 10 kann eine Befestigungseinrichtung 16 wie ein Pyramidenadapter angeordnet sein. Die Befestigungseinrichtung 16 kann grundsätzlich aber auch an anderen Stellen, insbesondere seitlich, des Hüllkörpers angeordnet sein. Die rechte Darstellung der Figur 6 zeigt ein Ausführungsbeispiel eines Hüllkörpers als Rahmenschaft mit einem Rahmen 23 und einer dorsalen Fassung mit einem distalen Endbereich 10 zum Aufnehmen eines Stumpfendes. Der feste Rahmen 23 umhüllt die Gliedmaße dabei nicht vollständig, der restliche Teil wird nur durch den mit Versteifungselementen 12 bestückten Hüllkörper ausgebildet und ist dadurch im mit Fluid befüllten Zustand flexibel.

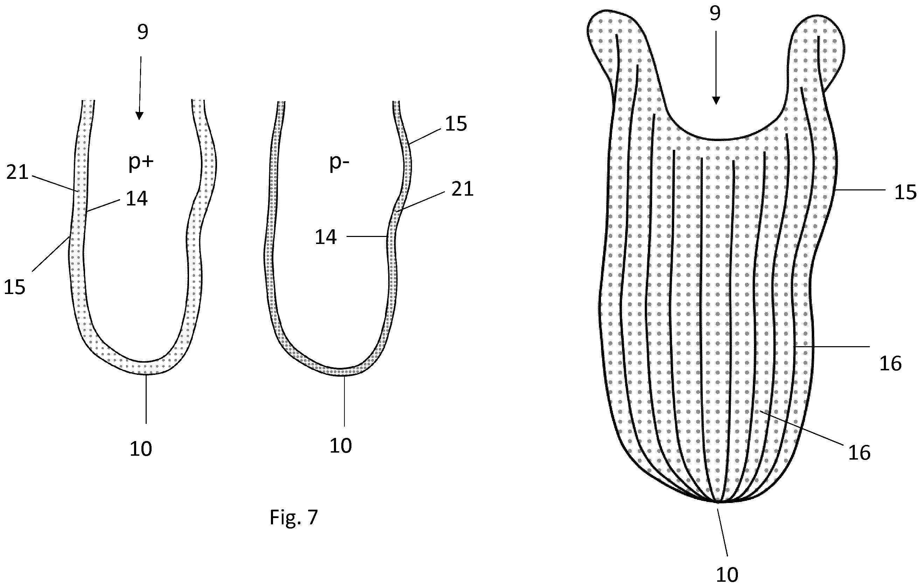

Figur 7 zeigt eine weitere Variante der Erfindung, wobei in der linken und mittleren Darstellung jeweils unterschiedliche Innendrücke in dem Volumen 21 vorhanden sind. Innerhalb des Volumens 21 ist ein Granulat angeordnet, das bei einer Evakuierung des Volumens 21, wie es in der mittleren Figur gezeigt ist, komprimiert wird, wodurch eine Relativbewegung der Granulatteilchen zueinander verhindert wird. Dadurch erhöht sich die Steifigkeit, da die Versteifungselemente in Gestalt der Granulatteilchen miteinander in Kontakt gebracht werden oder aufeinander gedrückt werden. In der Außenwand 15 oder an der Außenseite der Außenwand 15 können filamentartige Armierungen 16 angeordnet oder eingebettet sein, um eine Längsstabilität des Hüllkörpers 1 bereitzustellen. Eine Längung des Hüllkörpers 1 ist dann nicht mehr möglich oder noch eingeschränkt möglich, eine radiale Aufweitung bei Erhöhung des Druckes innerhalb des Volumens 21 ist dagegen möglich.

Die Filamentarmierung 16 kann auch radial durchgängig ausgebildet sein und Verbindungswände oder Zwischenwände zwischen der Innenwand 14 und der Außenwand 15 erzeugen, so dass eine Anzahl an voneinander separierten Einzelkammern über den Umfang verteilt vorliegt, so dass eine Vielzahl an Volumina innerhalb des Hüllkörpers 1 ausgebildet sind. Jedes Volumen kann mit einem Auslass oder einem Ventil versehen sein, um es separat zu befüllen oder zu entleeren oder mit einem Unterdruck zu versehen, wenn eine Vakuumpumpe angelegt wird.

Alle Ausführungsbeispiele der Figuren 5 - 7 können auch in den Hüllkörperbeispielen der Figuren 1 - 4 eingesetzt werden, insbesondere hinsichtlich der Versteifungselemente.

## Patentansprüche

1. Hüllkörper zur zumindest teilweisen Umhüllung einer Gliedmaße mit einem eingeschlossenen Volumen (21, 22) und einem Anschluss (3) zum Zuführen und Abführen von Fluid in das Volumen (21, 22) oder aus dem Volumen (21, 22), wobei der Hüllkörper (1) einen Innenumfang (4) und einen Außenumfang (5) ausbildet, **dadurch gekennzeichnet, dass** an dem Hüllkörper (1) Versteifungselemente (12, 13) angeordnet sind, die ausgebildet sind, durch ein Abführen von Fluid aus dem Volumen (21, 22) in Kontakt gebracht zu werden oder ihren Kontakt miteinander durch ein Abführen von Fluid zu verstärken.

2. Hüllkörper nach Anspruch 1, **dadurch gekennzeichnet, dass** die Versteifungselemente (12, 13) innerhalb des Volumens (21, 22) und/oder an dessen Außenumfang angeordnet sind.

3. Hüllkörper nach Anspruch 1 oder 2, **dadurch gekennzeichnet, dass** der Hüllkörper (1) zumindest zwei Wände (14, 15, 45) aufweist, zwischen denen das Volumen (21, 22) eingeschlossen ist und die Versteifungselemente (12, 13) auf der dem Volumen (21, 22) zugewandten Seite angeordnet und/oder in diese integriert sind und zugkraftübertragende Elemente (7) zwischen zumindest zwei Wänden (14, 15, 45) angeordnet sind.

4. Hüllkörper nach Anspruch 1 oder 2, **dadurch gekennzeichnet, dass** einander in dem Volumen (21, 22) gegenüberliegende Versteifungselemente (12, 13) oder nebeneinander angeordnete Versteifungselemente (12, 13) formschlüssig miteinander in Eingriff bringbar sind.

5. Hüllkörper nach einem der voranstehenden Ansprüche, **dadurch gekennzeichnet, dass** die Versteifungselemente (12, 13) an ihren Kontaktflächen einander korrespondierende Oberflächenstrukturen und/oder einen Reibungskoeffizienten µ ≥ 0,3 aufweisen.

6. Hüllkörper nach einem der voranstehenden Ansprüche, **dadurch gekennzeichnet, dass** nicht in Kontakt miteinander befindliche Versteifungselemente (12, 13) verlagerbar zueinander an oder in dem Hüllkörper (1) angeordnet sind.

7. Hüllkörper nach einem der voranstehenden Ansprüche, **dadurch gekennzeichnet, dass** der Hüllkörper (1) eine Innenwand (14) und eine Außenwand (15) aufweist, die das zumindest eine Volumen (21, 22) einschließen.

8. Hüllkörper nach einem der voranstehenden Ansprüche, **dadurch gekennzeichnet, dass** das zumindest eine Volumen (21, 22) miteinander verbundene Bereiche aufweist und/oder sich wendeiförmig in proximal-distal-Richtung erstreckt.

9. Hüllkörper nach einem der voranstehenden Ansprüche, **dadurch gekennzeichnet, dass** der Hüllkörper (1) mehrere über den Umfang verteilte und/oder radial hintereinander angeordnete, durch zumindest eine Zwischenwand (45) getrennte Volumina (21, 22) aufweist.

10. Hüllkörper nach Anspruch 9, **dadurch gekennzeichnet, dass** die Innenwand (14) und/oder Zwischenwand (45) und/oder Außenwand (15) faltbar und/oder elastisch ausgebildet ist.

11. Hüllkörper nach Anspruch 9 oder 10, **dadurch gekennzeichnet, dass** jedes Volumen einen Anschluss (3) zum Zuführen und Abführen von Fluid aufweist.

12. Hüllkörper nach einem der voranstehenden Ansprüche, **dadurch gekennzeichnet, dass** die Versteifungselemente (12, 13) als Streben, Platten, Granulat, Filamente, Gewebe, Gewirke, Gestricke und/oder strukturiertes Flächenmaterial ausgebildet sind.

13. Hüllkörper nach einem der voranstehenden Ansprüche, **dadurch gekennzeichnet, dass** der Innenumfang (4) des Hüllkörpers (1) sich bei einer Druckerhöhung des Volumens vergrößert.

14. Hüllkörper nach einem der voranstehenden Ansprüche, **dadurch gekennzeichnet, dass** der Hüllkörper (1) eine zumindest teilweise haftende Innenoberfläche (8) aufweist.

15. Hüllkörper nach einem der voranstehenden Ansprüche, **dadurch gekennzeichnet, dass** der Hüllkörper (1) als Prothesenliner, Bandage, Manschette, Kleidungsstück oder Schuh ausgebildet ist.

## Claims

1. An enveloping body for at least partially enveloping a limb, having an enclosed volume (21, 22) and a connector (3) for supplying and removing fluid to the volume (21, 22) and from the volume (21, 22), the enveloping body (1) forming an inner circumference (4) and an outer circumference (5), **characterized in that,** on the enveloping body (1), stiffening elements (12, 13) are arranged which are designed to be brought into contact as a result of removal of fluid from the volume (21, 22) or to strengthen their contact with one another as a result of removal of fluid.

2. The enveloping body as claimed in claim 1, **characterized in that** the stiffening elements (12, 13) are arranged inside the volume (21, 22) and/or on the outer circumference thereof.

3. The enveloping body as claimed in claim 1 or 2, **characterized in that** the enveloping body (1) has at least two walls (14, 15, 45) between which the volume (21, 22) is enclosed, and the stiffening elements (12, 13) are arranged on the side directed toward the volume (21, 22) and/or are integrated in said side and that elements (7) transmitting tensile force are arranged between at least two walls (14, 15, 45).

4. The enveloping body as claimed in claim 1 or 2, **characterized in that** stiffening elements (12, 13) lying opposite one another in the volume (21, 22) or stiffening elements (12, 13) arranged next to one another can be brought into form-fit engagement with one another.

5. The enveloping body as claimed in one of the preceding claims, **characterized in that,** on their contact faces, the stiffening elements (12, 13) have mutually corresponding surface structures and/or a coefficient of friction µ ≥ 0.3.

6. The enveloping body as claimed in one of the preceding claims, **characterized in that** stiffening elements (12, 13) that are not located in contact with one another are arranged to be movable relative to one another on or in the enveloping body (1).

7. The enveloping body as claimed in one of the preceding claims, **characterized in that** the enveloping body (1) has an inner wall (14) and an outer wall (15) which enclose the at least one volume (21, 22).

8. The enveloping body as claimed in one of the preceding claims, **characterized in that** the at least one volume (21, 22) has interconnected regions and/or extends helically in the proximal-distal direction.

9. The enveloping body as claimed in one of the preceding claims, **characterized in that** the enveloping body (1) has a plurality of volumes (21, 22) which are distributed about the circumference and/or arranged radially one after another and are separated by at least one intermediate wall (45).

10. The enveloping body as claimed in claim 9, **characterized in that** the inner wall (14) and/or intermediate wall (45) and/or outer wall (15) are/is foldable and/or elastic.

11. The enveloping body as claimed in claim 9 or 10, **characterized in that** each volume has a connector (3) for the supply and removal of fluid.

12. The enveloping body as claimed in one of the preceding claims, **characterized in that** the stiffening elements (12, 13) are designed as struts, plates, granules, filaments, wovens, knits and/or structured surface material.

13. The enveloping body as claimed in one of the preceding claims, **characterized in that** the inner circumference (4) of the enveloping body (1) increases as a pressure of the volume increases.

14. The enveloping body as claimed in one of the preceding claims, **characterized in that** the enveloping body (1) has an at least partially adhesive inner surface (8).

15. The enveloping body as claimed in one of the preceding claims, **characterized in that** the enveloping body (1) is designed as a prosthesis liner, bandage, cuff, item of clothing or shoe.

## Revendications

1. Corps enveloppant pour envelopper au moins partiellement un membre, présentant un volume enfermé (21, 22) et un raccord (3) pour amener et évacuer un fluide jusque dans le volume (21, 22) ou hors du volume (21, 22), le corps enveloppant (1) formant un pourtour intérieur (4) et un pourtour extérieur (5),
**caractérisé en ce que** des éléments de rigidification (12, 13) sont disposés sur le corps enveloppant (1) et sont réalisés pour être mis en contact par une évacuation de fluide hors du volume (21, 22) ou pour renforcer leur contact mutuel par une évacuation de fluide.

2. Corps enveloppant selon la revendication 1,
**caractérisé en ce que** les éléments de rigidification (12, 13) sont disposés à l'intérieur du volume (21, 22) et/ou sur son pourtour extérieur.

3. Corps enveloppant selon la revendication 1 ou 2,
**caractérisé en ce que** le corps enveloppant (1) présente au moins deux parois (14, 15, 45) entre lesquelles le volume (21, 22) est enfermé, et les éléments de rigidification (12, 13) sont disposés sur la face tournée vers le volume (21, 22) et/ou sont intégrés dans celle-ci, et des éléments (7) de transmission de force de traction sont disposés entre au moins deux parois (14, 15, 45).

4. Corps enveloppant selon la revendication 1 ou 2,
**caractérisé en ce que** des éléments de rigidification (12, 13) opposés les uns aux autres dans le volume (21, 22) ou des éléments de rigidification (12, 13) disposés les uns à côté des autres peuvent être amenés en engagement les uns avec les autres par coopération de forme.

5. Corps enveloppant selon l'une des revendications précédentes,
**caractérisé en ce que** les éléments de rigidification (12, 13) présentent sur leurs surfaces de contact des structures de surface correspondantes et/ou un coefficient de frottement µ ≥ 0,3.

6. Corps enveloppant selon l'une des revendications précédentes,
**caractérisé en ce que** des éléments de rigidification (12, 13) qui ne sont pas en contact les uns avec les autres sont disposés sur ou dans le corps enveloppant (1) de manière à pouvoir être déplacés les uns par rapport aux autres.

7. Corps enveloppant selon l'une des revendications précédentes,
**caractérisé en ce que** le corps enveloppant (1) présente une paroi intérieure (14) et une paroi extérieure (15) qui enferment ledit au moins un volume (21, 22).

8. Corps enveloppant selon l'une des revendications précédentes,
**caractérisé en ce que** ledit au moins un volume (21, 22) présente des zones reliées entre elles et/ou s'étend en forme d'hélice dans la direction proximale-distale.

9. Corps enveloppant selon l'une des revendications précédentes,
**caractérisé en ce que** le corps enveloppant (1) présente plusieurs volumes (21, 22) répartis sur la périphérique et/ou disposés radialement les uns derrière les autres et séparés par au moins une paroi intermédiaire (45).

10. Corps enveloppant selon la revendication 9,
**caractérisé en ce que** la paroi intérieure (14) et/ou la paroi intermédiaire (45) et/ou la paroi extérieure (15) est réalisée de manière pliable et/ou élastique.

11. Corps enveloppant selon la revendication 9 ou 10,
**caractérisé en ce que** chaque volume présente un raccord (3) pour amener et évacuer un fluide.

12. Corps enveloppant selon l'une des revendications précédentes,
**caractérisé en ce que** les éléments de rigidification (12, 13) sont réalisés sous forme d'entretoises, de plaques, de granulés, de filaments, de tissus, de maillages, de tricots et/ou de matériau plat structuré.

13. Corps enveloppant selon l'une des revendications précédentes,
**caractérisé en ce que** le pourtour intérieur (4) du corps enveloppant (1) s'agrandit lors d'une augmentation de la pression du volume.

14. Corps enveloppant selon l'une des revendications précédentes,
**caractérisé en ce que** le corps enveloppant (1) présente une surface intérieure (8) au moins partiellement adhérente.

15. Corps enveloppant selon l'une des revendications précédentes,
**caractérisé en ce que** le corps enveloppant (1) est réalisé sous forme de doublure prothétique, de bandage, de manchon, de vêtement ou de chaussure.
